# EUROPEAN PATENT APPLICATION

(11) **EP 3 664 058 A1**
(43) Date of publication of application: **10.06.2020**
(21) Application number: 18840733.2
(22) Date of filing: 25.07.2018
(51) Int. Cl.: G08C 15/06, G06Q 50/10, G08C 15/00

(54) **SENSOR MANAGEMENT UNIT, SENSOR APPARATUS, SENSING DATA PROVISION METHOD, AND SENSING DATA PROVISION PROGRAM**

(30) Priority: 01.08.2017 JP 2017149191
(71) Applicant: Omron Corporation, Kyoto-shi, Kyoto 600-8530 (JP)
(72) Inventor: ODA, Toshihiko, Kyoto 619-0283 (JP); YAMATO, Tetsuji, Kyoto 619-0283 (JP)
(74) Representative: Hoffmann Eitle
(86) International application number: PCT/JP2018/027820
(87) International publication number: WO 2019/026708

(57) **Abstract**

A sensor management unit includes a sensing data obtaining unit that obtains sensing data generated by a sensor, an output determination unit that determines whether the sensing data is allowed to be output to a user based on a data catalog storing an attribute of the sensing data and storing a first item allowed to be output to the user, and an output unit that outputs the sensing data to the user in accordance with a result of the determination by the output determination unit.

## Description

### FIELD

The present invention relates to a technique for distributing sensing data between a provider and a user.

### BACKGROUND

A sensor network system has been developed to distribute sensing data sensed by sensing devices between a provider and a user (refer to, for example, Patent Literature 1). Each sensing device is a sensor, or a device connectable to multiple sensors. The sensing data represents the monitoring characteristics for a monitoring target sensed by the sensing device. Monitoring targets include abstract objects representing real-world phenomena (e.g., persons, objects, and events). The monitoring characteristics refer to the attributes of a monitoring target to be monitored by a sensor. Monitoring targets include an adult, a household, and an automobile. The monitoring characteristics for an adult include a maximum blood pressure, a minimum blood pressure, and a pulse. The monitoring characteristics for a household include the amounts of electricity use, gas use, and water use. The monitoring characteristics for a vehicle include the position, speed, and fuel consumption.

A provider registers, with a network server, a sensing device, and also sensor metadata for sensing data to be sensed and provided by the sensing device. A user registers, with the network server, an application that uses sensing data, and also application metadata for sensing data to be used by the application. The sensor metadata is information about a sensing device, and also about the attributes of sensing data to be sensed and provided by the sensing device. The application metadata is information about an application, and also about the attributes of sensing data to be used by the application.

The network server performs matching using the sensor metadata and the application metadata, and retrieves a sensing device that provides sensing data satisfying a request from the application. The network server transmits a data flow control command to a sensor management device that manages the retrieved sensing device. The data flow control command causes a data provider (sensing device) to distribute sensing data to a data user (application).

### CITATION LIST

### PATENT LITERATURE

Patent Literature 1: Japanese Patent No. 5445722

### SUMMARY

### TECHNICAL PROBLEM

However, a provider data catalog (corresponding to the sensor metadata described in Patent Literature 1) stores information about a sensor and about the attributes of the sensing data obtained by the sensor, and can include inappropriate information due to misunderstandings or oversights by the provider. When the provider data catalog registered with the network server includes inappropriate information unintended by the provider, sensing data unintended by the provider can be distributed to the user.

One or more aspects of the present invention are directed to a technique for reducing sensing data unintended by the provider being distributed to the user.

### SOLUTION TO PROBLEM

In response to the above issue, a sensor management unit according to one or more aspects of the present invention has the structure described below.

A sensing data obtaining unit obtains sensing data generated by a sensor. An output determination unit determines whether the sensing data is allowed to be output to a user based on a data catalog storing an attribute of the sensing data and storing a first item allowed to be output to the user. An output unit outputs the sensing data to the user in accordance with a result of the determination by the output determination unit.

This structure avoids setting sensing data unintended for distribution to the user in a sensor network system as an item allowed to be output to the user, and thus reduces sensing data unintended by the provider being distributed to the user.

The structure may further include an edit unit that receives an edit to an item allowed to be output to the user. The edit unit thus allows an edit indicating whether to set each item of sensing data (e.g., a maximum blood pressure, a minimum blood pressure, and a pulse) as an item allowed to be output to the user.

The edit unit may also receive an edit indicating whether personal information about the provider is to be converted into anonymized information before the information is output to the user.

### ADVANTAGEOUS EFFECTS

The sensor management unit according to the above aspect of the present invention reduces sensing data unintended by the provider of the sensing data being distributed to the user.

### BRIEF DESCRIPTION OF THE DRAWINGS

Fig. 1 is a schematic diagram of a sensing data distribution system.
Fig. 2 is a block diagram of a gateway (GW) terminal showing its main components.
Fig. 3 is a schematic diagram of a provider data catalog.
Fig. 4 is a diagram of an information collection table.
Fig. 5 is a flowchart showing a process for registering the sensing device performed by the GW terminal according to an embodiment.
Fig. 6 is a flowchart showing a process for generating the provider data catalog performed by the GW according to the embodiment.
Figs. 7A and 7B are diagrams of example screens for editing the data catalog performed by the GW terminal.
Fig. 8A is a diagram of an example screen for editing and generating a first data catalog performed by the GW terminal, and Fig. 8B is a diagram of an example screen for editing and generating a second data catalog performed by the GW terminal.
Fig. 9 is a diagram of an example screen on the GW terminal for editing and generating the terms of sales for sensing data.
Fig. 10 is a flowchart showing a process for collecting data performed by the GW terminal.
Fig. 11 is a flowchart showing a process for providing sensing data performed by the GW terminal.
Fig. 12 is a block diagram of a sensor device showing its main components.

### DETAILED DESCRIPTION

One or more embodiments of the present invention will now be described.

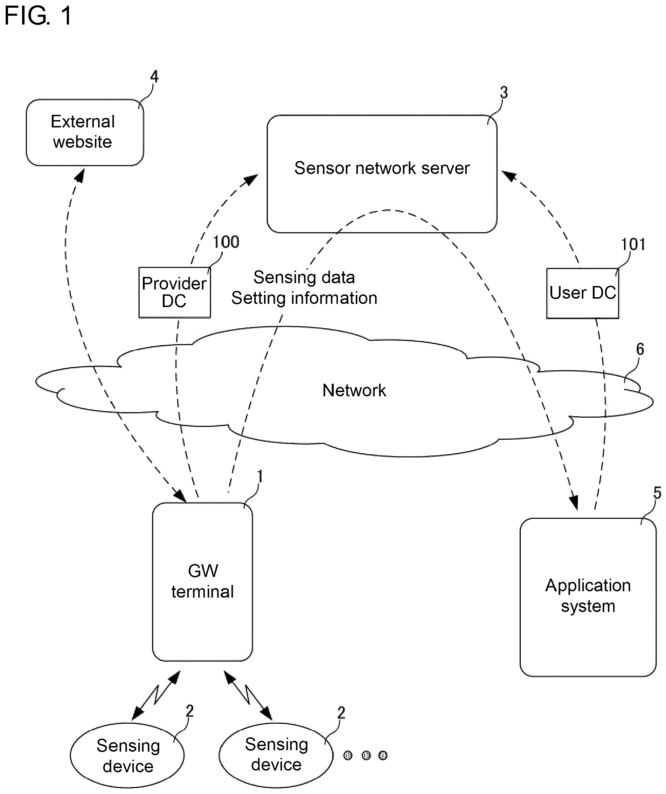

A sensing data distribution system will be described first. Fig. 1 is a schematic diagram of the sensing data distribution system according to an embodiment. The sensing data distribution system according to the embodiment includes a gateway (GW) terminal 1, sensing devices 2, a sensor network server 3, an external website 4, and an application system 5. The sensing data distribution system distributes sensing data between a provider and a user. The GW terminal 1 corresponds to a sensor management unit in an aspect of the present invention. Each sensing device 2 is a sensor, or a device connectable to multiple sensors. The sensing device 2 corresponds to a sensor in an aspect of the present invention.

In the example sensing data distribution system described herein, the GW terminal 1 transmits, in addition to sensing data, setting information of setting items (described later) to the application system 5 as the user. In some embodiments, the sensor management unit may not transmit setting information of setting items to the application system 5 as the user (in other words, may transmit sensing data to the application system 5 without transmitting the setting information of the setting items).

The GW terminal 1 and the sensing devices 2 are components of the provider providing sensing data. The application system 5 is a component of the user using sensing data. The sensor network server 3 defines a sensing data distribution market that serves as a marketplace for distributing sensing data on the Internet, or specifically a sensing data trading market (SDTM).

The external website 4 contains web pages published on the Internet. In this example, the external website 4 is a website for selling products or delivering services to registered users. The external website 4 stores information about each registered user, such as the sales history of products, the delivery history of services, and personal information about the registered user (e.g., the name, age, sex, and address). The sales history of products and the delivery history of services stored in the external website 4 for each registered user will be collectively referred to as the use history. The GW terminal 1, the sensor network server 3, the he external website 4, and the application system 5 are connected through a network 6 to allow data communication between them. The sensing devices 2 are connected to the GW terminal 1 with wires or wirelessly.

The external website 4 does not disclose information about the registered users (the use history and the personal information) to third parties. More specifically, the external website 4 identifies each registered user using a user identification code (ID) and an identification verification code (a password or a PW), and allows disclosure of the use history and personal information about the registered user exclusively to a successfully identified registered user.

The provider transmits a provider data catalog 100 (provider data catalog or DC 100), which is associated with sensing data to be traded (to sell) in the SDTM, and registers the provider data catalog 100 with the sensor network server 3. The provider data catalog 100 stores sensing data and setting items. In this example, the provider data catalog 100 is a pair of data catalogs including a first data catalog 100a storing attribute information about sensing data and a second data catalog 100b storing one or more setting items. The provider data catalog 100 will be described in detail later.

The setting items are items associated with a monitoring target for which sensing data is to be provided. More specifically, the setting items are metadata about the sensing data. As described above, monitoring targets include abstract objects representing real-world phenomena (e.g., persons, objects, and events). When the monitoring target is a person, the setting items include the height, age, sex, lifestyle pattern, and the history of taking medicines (medication history) of the person. When the monitoring target is a household, the setting items include the family members, lifestyle pattern, and address of the household. When the monitoring target is a vehicle, the setting items include the width, length, height, and displacement of the vehicle. The setting information is information (data) representing the values or actual data of the setting items.

The user transmits a user data catalog 101 (user DC 101), which is associated with sensing data to be traded (to purchase) in the SDTM, and registers the user data catalog 101 with the sensor network server 3. The user data catalog 101 stores attribute information about sensing data. The user data catalog 101 may or may not store setting items.

The sensor network server 3 performs matching for retrieving a provider that provides sensing data satisfying the user data catalog 101 based on the registered provider data catalog 100 and the user data catalog 101. When the user data catalog 101 does not store setting items, the matching is performed to retrieve a provider that can provide the sensing data satisfying the user data catalog 101 without using setting items. When the user data catalog 101 stores setting items, the matching is performed to retrieve a provider that can provide the sensing data satisfying the user data catalog 101 using the setting items.

The first data catalog 100a storing the attribute information about the sensing data may be used as the provider data catalog 100 registered with the sensor network server 3 (the second data catalog 100b storing setting items may not be registered with the sensor network server 3 as the provider data catalog 100). In this case, the sensor network server 3 performs matching without using setting items.

In response to the data flow control command resulting from the matching, the provider transmits, to the user, setting information of setting items together with the sensing data. The data flow control command is generated by any of, for example, the sensor network server 3, the provider, and the user depending on the result of the matching. More specifically, the device that generates the data flow control command is not limited to a specific device.

The sensor network server 3 is connectable to multiple providers (the GW terminal 1 and the sensing devices 2) with a network 6. The sensor network server 3 is connectable to multiple users (application systems 5) with the network 6. Fig. 1 shows a single provider and a single user. Sensing data and setting information may be transmitted from the provider to the user through the sensor network server 3 as shown in Fig. 1, or may be transmitted from the provider to the user directly without using the sensor network server 3.

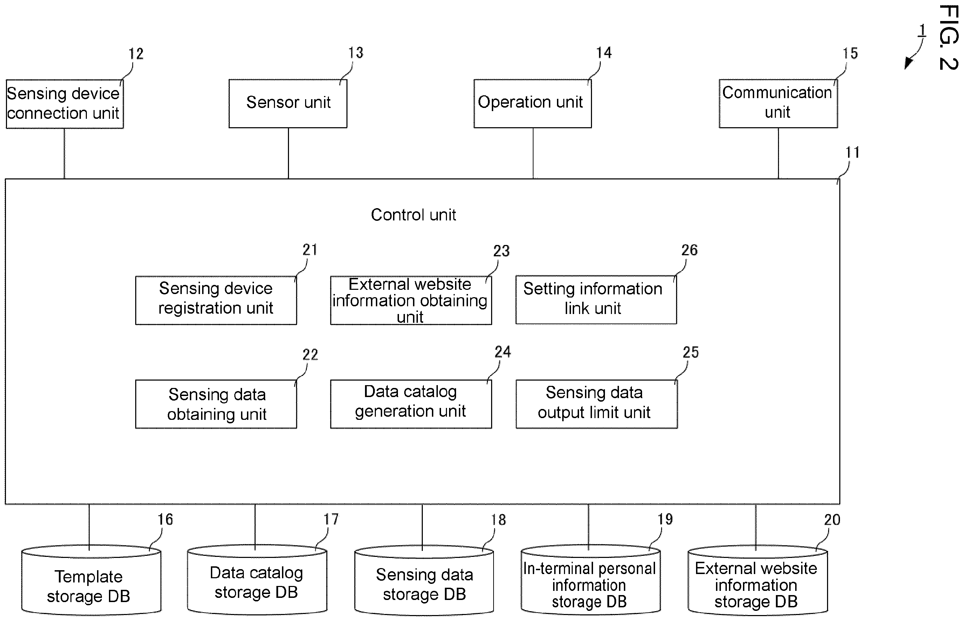

Fig. 2 is a block diagram of the GW terminal 1 showing its main components. The GW terminal 1 includes a control unit 11, a sensing device connection unit 12, a sensor unit 13, an operation unit 14, a communication unit 15, a template storage database (DB) 16, a data catalog storage database (DB) 17, a sensing data storage database (DB) 18, an in-terminal personal information storage database (DB) 19, and an external website information storage database (DB) 20. The GW terminal 1 may be a personal computer (PC), a mobile terminal such as a smartphone or a tablet, or may be any other information processing device. The GW terminal 1 is a smartphone in this embodiment.

The control unit 11 controls the operation of each main component of the GW terminal 1. As shown in Fig. 2, the control unit 11 includes a sensing device registration unit 21, a sensing data obtaining unit 22, an external website information obtaining unit 23, a data catalog generation unit 24, a sensing data output limit unit 25, and a setting information link unit 26. The sensing device registration unit 21, the sensing data obtaining unit 22, the external website information obtaining unit 23, the data catalog generation unit 24, the sensing data output limit unit 25, and the setting information link unit 26 included in the control unit 11 will be described in detail later.

The sensing device connection unit 12 connects the sensing devices 2 with wires or wirelessly. The sensing device connection unit 12 functions as an interface for controlling input and output of data with the sensing devices 2.

The sensor unit 13 includes sensors incorporated in the GW terminal 1, such as a global positioning system (GPS) sensor, a magnetic sensor, and a temperature sensor. In another example, the GW terminal 1 incorporates no sensor (does not include the sensor unit 13).

The sensing devices 2 and the sensors included in the sensor unit 13 sense the characteristics of monitoring targets.

The operation unit 14 receives an input operation performed by an operator operating the GW terminal 1. In this embodiment, the operation unit 14 includes a display and a touch panel attached to the screen of the display. The operation unit 14 also controls screens to appear on the display (e.g., screens to be a user interface).

The communication unit 15 controls data communication with external devices through the network 6. The sensor network server 3, the external website 4, and the application system 5 shown in Fig. 1 herein correspond to the external devices. The external devices are not limited to the sensor network server 3, the external website 4, and the application system 5. The communication unit 15 corresponds to an output unit in an aspect of the present invention.

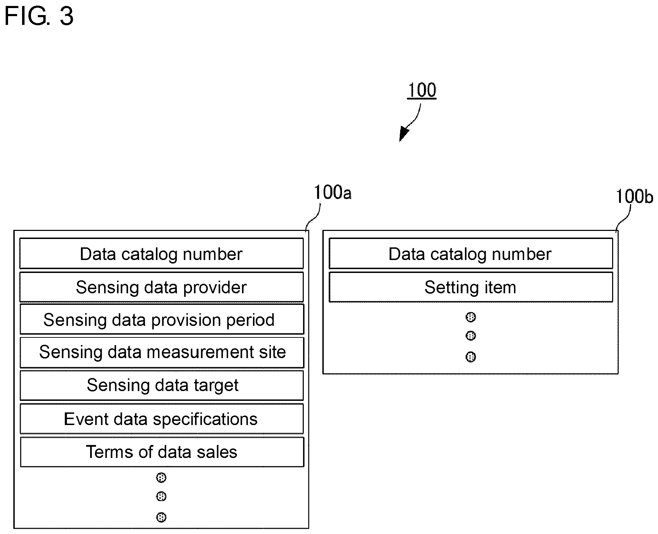

The template storage DB 16 stores templates for the provider data catalog 100. The provider data catalog 100 will now be described. Fig. 3 is a diagram of the provider data catalog 100. As described above, the provider data catalog 100 is a pair of data catalogs including the first data catalog 100a storing attribute information about sensing data and the second data catalog 100b storing one or more setting items.

The first data catalog 100a mainly stores a data catalog number, a sensing data provider, a sensing data provision period, a sensing data measurement site, a sensing data target, event data specifications, and the terms of a data sales contract. The data catalog number is used to identify the provider data catalog 100. The sensing data provider is attribute information representing the organization (individual or business entity) providing the sensing data. The sensing data provision period is attribute information representing the period for providing the sensing data. The sensing data measurement site is attribute information representing the site at which the monitoring target undergoes sensing. The sensing data target is attribute information representing the monitoring target and the monitoring characteristics. The event data specifications are attribute information representing an event condition. The terms of the data sales contract are attribute information associated with the sensing data trading.

The second data catalog 100b mainly stores a data catalog number and setting items. The data catalog number is used to identify the provider data catalog 100. The first data catalog 100a and the second data catalog 100b are associated with each other using the data catalog number. The setting items are, as described above, items associated with the monitoring target for which sensing data is to be provided.

The provider data catalog 100 may include a single data catalog storing both the attribute information about the sensing data and the setting items.

The template storage DB 16 stores the templates for the first data catalog 100a and the second data catalog 100b.

The data catalog storage DB 17 is a storage for storing the provider data catalog 100. The data catalog storage DB 17 stores at least the provider data catalog 100 registered with the sensor network server 3. More specifically, the provider data catalog 100 registered with the sensor network server 3 is stored in the data catalog storage DB 17.

The data catalog storage DB 17 may also store a provider data catalog 100 unregistered with the sensor network server 3.

The sensing data storage DB 18 stores sensing data representing the characteristics of the monitoring target sensed by the sensing devices 2 and the sensors included in the sensor unit 13.

The in-terminal personal information storage DB 19 stores the personal information registered with the GW terminal 1 by the owner of the GW terminal 1 (also the provider of sensing data in this example). The personal information includes, for example, the name, height, age, sex, address book, and ID and PW for the external website 4 being used.

The external website information storage DB 20 stores, for example, the personal information and the use history of the owner of the GW terminal 1 obtained through access to the external website 4.

At least two of the template storage DB 16, the data catalog storage DB 17, the sensing data storage DB 18, the in-terminal personal information storage DB 19, and the external website information storage DB 20 may be combined into an integrated database. For example, the template storage DB 16 and the data catalog storage DB 17 may be combined into a single database. The in-terminal personal information storage DB 19 and the external website information storage DB 20 may be combined into a single database. The template storage DB 16, the data catalog storage DB 17, the sensing data storage DB 18, the in-terminal personal information storage DB 19, and the external website information storage DB 20 may be combined into a single database.

The sensing device registration unit 21, the sensing data obtaining unit 22, the external website information obtaining unit 23, the data catalog generation unit 24, the sensing data output limit unit 25, and the setting information link unit 26 included in the control unit 11 will be now be described.

The sensing device registration unit 21 registers the device identifier of the sensing device 2 with the GW terminal 1. The GW terminal 1 can register multiple sensing devices 2. The GW terminal 1 cannot be used to trade, in the SDTM, sensing data sensed by an unregistered sensing device 2. In other words, only sensing data sensed by sensing devices 2 registered with the GW terminal 1 can be traded in the SDTM. The device identifier refers to information identifying the type of the sensing device 2.

The provider can choose not to trade sensing data sensed by a sensing device 2 registered with the GW terminal 1 in the SDTM.

The sensing data obtaining unit 22 obtains sensing data sensed by a sensing device 2 from the sensing device 2 connected through the sensing device connection unit 12. The sensing data obtaining unit 22 also obtains the sensing data sensed by the sensors included in the sensor unit 13. The sensing data obtaining unit 22 stores the obtained sensing data into the sensing data storage DB 18. The sensing data obtaining unit 22 corresponds to a sensing data obtaining unit in an aspect of the present invention.

The external website information obtaining unit 23 accesses the external website 4, and obtains, for example, the personal information and the use history of the owner of the GW terminal 1 stored in the external website 4. When accessing the external website 4, the external website information obtaining unit 23 uses the ID and the PW for the external website 4 registered into the in-terminal personal information storage DB 19. The external website information obtaining unit 23 stores, for example, the obtained personal information and use history of the owner of the GW terminal 1 into the external website information storage DB 20.

The data catalog generation unit 24 generates the provider data catalog 100 to be registered with the sensor network server 3. The data catalog generation unit 24 generates the provider data catalog 100 using the templates for the first data catalog 100a and the second data catalog 100b stored in the template storage DB 16. The data catalog generation unit 24 receives an editing operation performed by the operator (provider), and generates the provider data catalog 100. The operator edits the provider data catalog 100 through the operation unit 14. The data catalog generation unit 24 corresponds to an edit unit in an aspect of the present invention.

The sensing data output limit unit 25 filters and classifies sensing data sensed by the sensing device 2 or a sensor incorporated in the GW terminal 1 and obtained by the sensing data obtaining unit 22. Through filtering, the sensing data is classified into data items allowed to be transmitted to an external device (user) through the communication unit 15, and data items not allowed to be transmitted to the external device. More specifically, the sensing data output limit unit 25 determines whether each item of sensing data is allowed to be transmitted to an external device (user) through the communication unit 15.

The setting information link unit 26 links, with the sensing data filtered by the sensing data output limit unit 25 and to be transmitted to an external device (user), the setting information of the setting items associated with the sensing data. The setting information of the setting items is stored in the in-terminal personal information storage DB 19 and the external website information storage DB 20. In this embodiment, the sensing data output limit unit 25 and the setting information link unit 26 correspond to an output determination unit in an aspect of the present invention.

The control unit 11 in the GW terminal 1 includes a hardware central processing unit (CPU), a memory, and other electronic circuits. The hardware CPU functions as the sensing device registration unit 21, the sensing data obtaining unit 22, the external website information obtaining unit 23, the data catalog generation unit 24, the sensing data output limit unit 25, and the setting information link unit 26 described above. The memory has an area for expanding a sensing data provision program according to one or more embodiments of the present invention and an area for temporarily storing data generated by executing the sensing data provision program. The control unit 11 may be a large scale integrated circuit (LSI) integrating, for example, a hardware CPU and a memory.

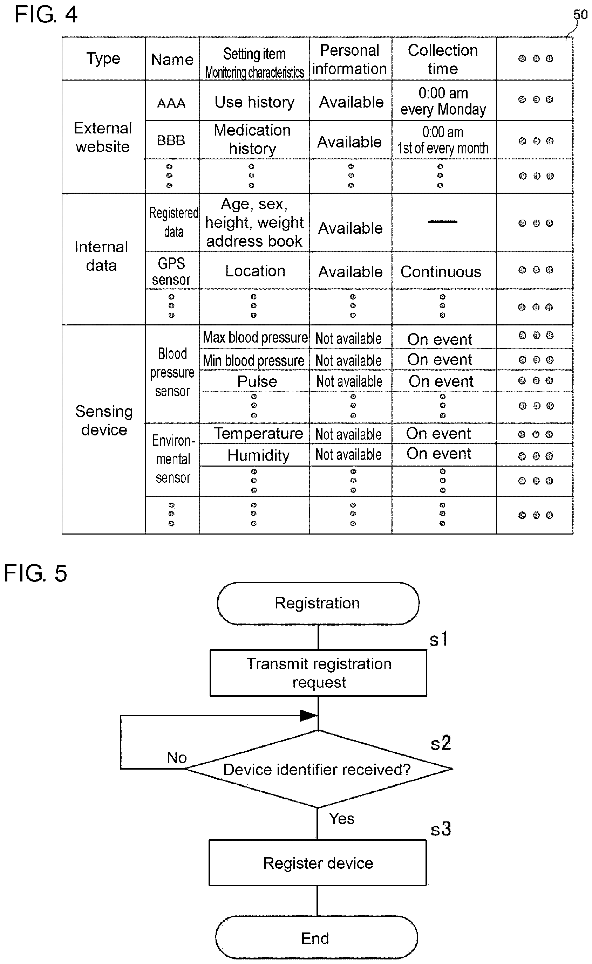

The control unit 11 included in the GW terminal 1 stores an information collection table 50 shown in Fig. 4 in the memory (not shown) included in the control unit 11. The information collection table 50 stores, for example, the types of information obtained from external websites 4, the sensing devices 2, and the sensors included in the sensor unit 13 (sensing data and information from external websites) and the times for obtaining such information. For example, for an external website 4 named AAA shown in Fig. 4, the use history is obtained at 0:00 am every Monday (the external website 4 named AAA is, for example, a website for selling products). For an external website 4 named BBB, the medication history is obtained at 0:00 am on the first day of every month (the external website 4 named BBB is, for example, a website for managing a prescription record).

The operation of the GW terminal 1 will now be described. The processes performed by the GW terminal 1 for registering a sensing device 2 will be described first. Fig. 5 is a flowchart showing a process for registering the sensing device 2 performed by the GW terminal 1 according to the embodiment.

The sensing device registration unit 21 in the GW terminal 1 transmits a registration request to the sensing device 2 connected to the sensing device connection unit 12 (s1). When receiving the registration request transmitted from the GW terminal 1, the sensing device 2 transmits its device identifier to the GW terminal 1. The sensing device 2 stores the device identifier in its memory.

When receiving the device identifier transmitted from the sensing device 2 (s2), the sensing device registration unit 21 performs device registration to store the received device identifier in a memory (s3), and ends the processing.

In the registration process, the sensing device registration unit 21 may obtain the template for the first data catalog 100a from the sensing device 2 that stores the template for the first data catalog 100a. In this case, the sensing device registration unit 21 stores the obtained template for the first data catalog 100a into the template storage DB 16 in a manner associated with the device identifier. For a sensing device 2 that stores a uniform resource locator (URL) of the Internet site storing the template for the first data catalog 100a, the sensing device registration unit 21 may obtain the URL, and access the obtained URL to obtain the template for the first data catalog 100a. For a sensing device 2 that stores a digital object identifier (DOI) identifying the template for the first data catalog 100a, the sensing device registration unit 21 may obtain the DOI, and access the obtained DOI to obtain the template for the first data catalog 100a.

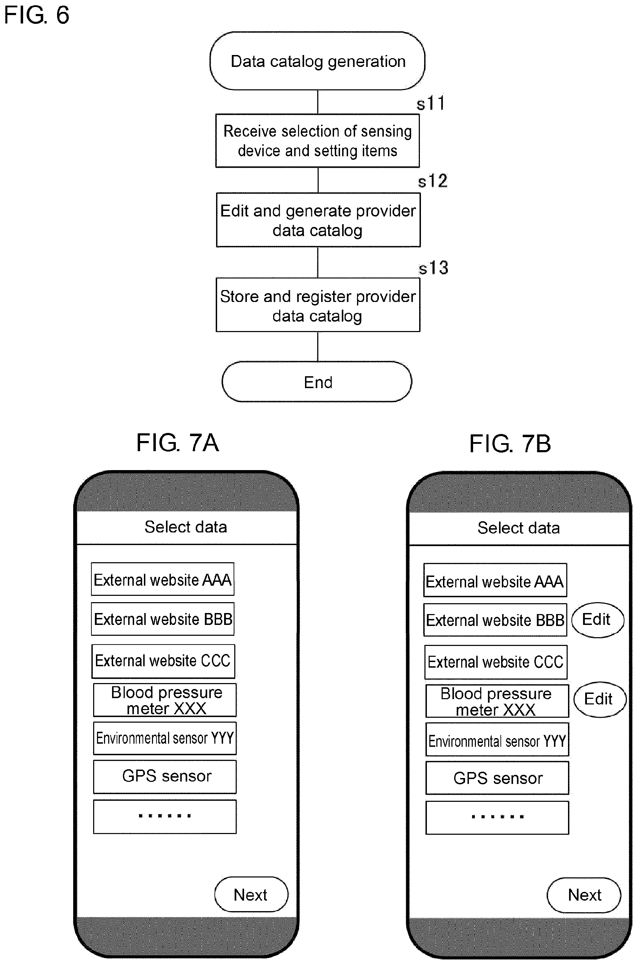

A process performed by the GW terminal 1 for generating the provider data catalog 100 will now be described. Fig. 6 is a flowchart showing the process for generating the provider data catalog 100 performed by the GW terminal 1 according to the embodiment. The GW terminal 1 receives selection of the sensing device 2 and the setting items for which the data catalog generation unit 24 generates the provider data catalog 100 this time (s11). The data catalog generation unit 24 shows, for example, the screen shown in Fig. 7A on the display, and receives selection of a sensing device 2 and setting items (e.g., an external website 4) from the operator. The data catalog generation unit 24 shows an edit button for each item selected in s11 (refer to Fig. 7B). More specifically, the items shown with edit buttons in Fig. 7B are the selected items. In Fig. 7B, an external website BBB and a blood pressure meter XXX are selected. The external website BBB is an external website 4. The blood pressure meter XXX is a sensing device 2.

In response to an operation on the edit button for one of the items selected in s11, the data catalog generation unit 24 edits and generates the data catalog associated with the selected item (s12). In s12, when the selected item is a sensing device 2 or an internal sensor incorporated in the sensor unit 13, the data catalog generation unit 24 edits and generates the first data catalog 100a. When the selected item is a setting item stored in the in-terminal personal information storage DB 19 or the external website information storage DB 20, the data catalog generation unit 24 edits and generates the second data catalog 100b.

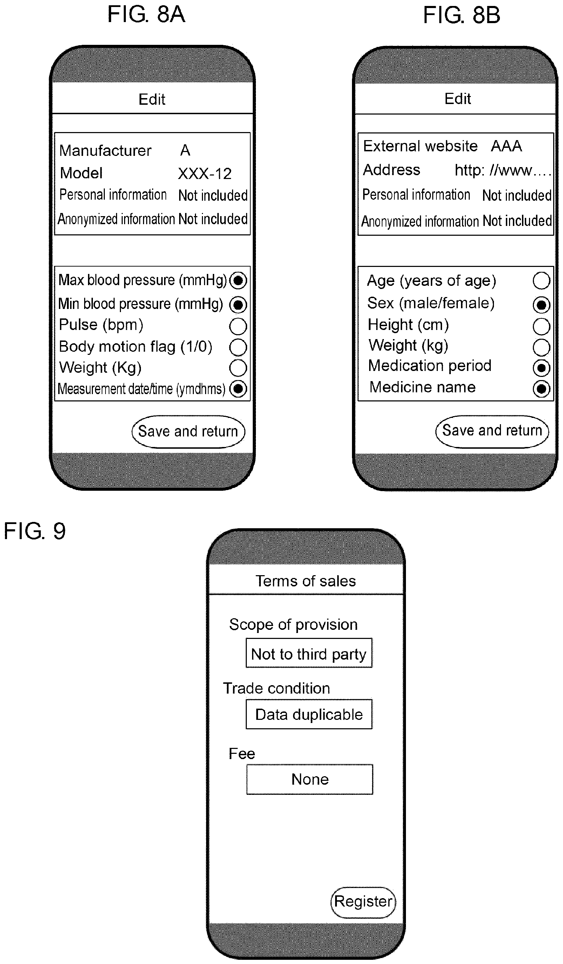

The data catalog generation unit 24 reads the template for the selected item from the template storage DB 16, and shows the screen shown in Fig. 8A or 8B on the display to receive an edit operation from the operator. Fig. 8A is an example screen that appears when the edit button for the blood pressure meter XXX is operated. Fig. 8B is an example screen that appears when the edit button for the external website BBB is operated. On the screens shown in Figs. 8A and 8B, for example, the operator selects data items to be traded in the SDTM. The screens shown in Figs. 8A and 8B allow selection of the data items to be provided to the user. On the screen shown in Fig. 8A, the items of sensing data selected for provision to the user are the maximum blood pressure, minimum blood pressure, and measurement date and time (items of sensing data unselected for provision to the user are the pulse, body motion flag, and weight). On the screen shown in Fig. 8B, the setting items selected as setting information for provision to the user are the sex, medication period, and medicine name (items of data unselected for provision to the user are the age, height, and weight).

The screens shown in Figs. 8A and 8B also allow setting to or not to provide the personal information about the provider or anonymized information obtained by anonymizing the personal information together with the data to be provided to the user.

In response to a press on the save and return button appearing on the screen shown in Fig. 8A or 8B, the data catalog generation unit 24 returns to the screen shown in Fig. 7B. In the manner described above, the data catalog generation unit 24 receives, through the screen shown in Fig. 8A or 8B, an edit to the data catalog (the first data catalog 100a or the second data catalog 100b) associated with the item selected on the screen shown in Fig. 7B.

In response to a press on the next button appearing on the screen shown in Fig. 7B, the data catalog generation unit 24 shows the screen shown in Fig. 9 on the display, and receives, from the operator, an edit operation on the terms of data sales. Through the screen shown in Fig. 9, the data catalog generation unit 24 receives edits to, for example, the scope of provision, a trade condition, and the fee of the data to be traded in the SDTM.

In response to a press on the register button appearing on the screen shown in Fig. 9, the data catalog generation unit 24 determines that the operator has completed the edit operations of the provider data catalog 100, and generates the provider data catalog 100 by associating the edited first data catalog 100a and second data catalog 100b with each other. The data catalog generation unit 24 assigns a data catalog number to the edited first data catalog 100a and second data catalog 100b to associate them with each other.

The data catalog generation unit 24 stores the provider data catalog 100 generated in s12 into the data catalog storage DB 17, and transmits the provider data catalog 100 to the sensor network server 3 to register the provider data catalog 100 with the sensor network server 3 (s13).

As described above, the GW terminal 1 according to the embodiment generates the provider data catalog 100 including the associated first data catalog 100a and second data catalog 100b, and registers the provider data catalog 100 with the sensor network server 3.

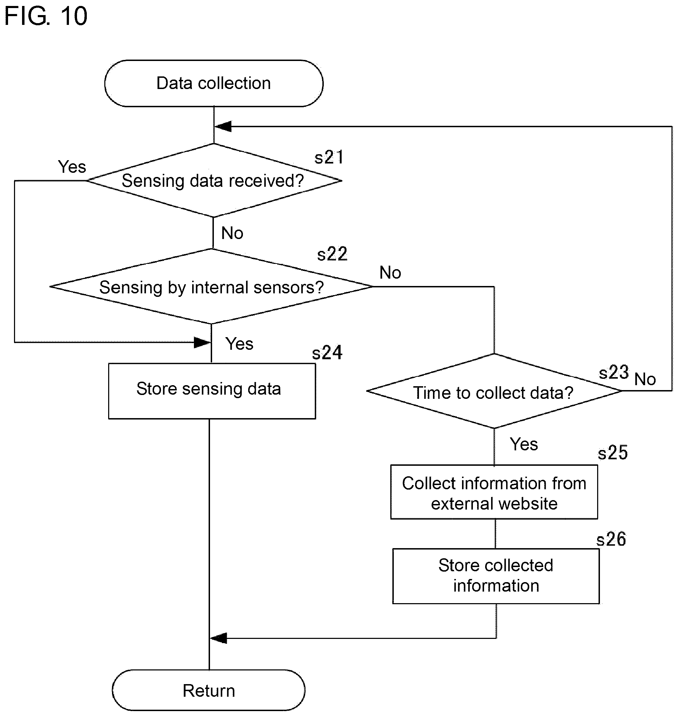

A process for collecting data performed by the GW terminal 1 will now be described. Fig. 10 is a flowchart showing a process for collecting data. When the sensing data obtaining unit 22 included in the GW terminal 1 receives, through the sensing device connection unit 12, sensing data representing the characteristics of a monitoring target sensed by a connected sensing device 2, the sensing data obtaining unit 22 stores the received sensing data into the sensing data storage DB 18 (s21 and s24). When an internal sensor incorporated in the sensor unit 13 senses a characteristic of a monitoring target, the sensing data obtaining unit 22 included in the GW terminal 1 stores the sensing data into the sensing data storage DB 18 (s22 and s24).

When the external website information obtaining unit 23 included in the GW terminal 1 determines that the current time is the information collection time to obtain setting information of the setting items from any external website 4 by referring to the information collection table 50 shown in Fig. 4, the external website information obtaining unit 23 accesses the external website 4 and obtains the setting information of the setting items (s23 and s25). The external website information obtaining unit 23 uses the ID and the PW for the external website 4 stored in the in-terminal personal information storage DB 19 to obtain the setting information of the setting items. The external website information obtaining unit 23 stores the obtained setting information of the setting items into the external website information storage DB 20 (s26).

The GW terminal 1 repeats the processing in s21 to s26 described above. In this manner, the GW terminal 1 stores, into the sensing data storage DB 18, the sensing data representing the characteristics of a monitoring target sensed by the sensing device 2 or the internal sensors incorporated in the sensor unit 13. The GW terminal 1 also stores, into the external website information storage DB 20, the setting information of the setting items obtained by the external website information obtaining unit 23 accessing the external website 4.

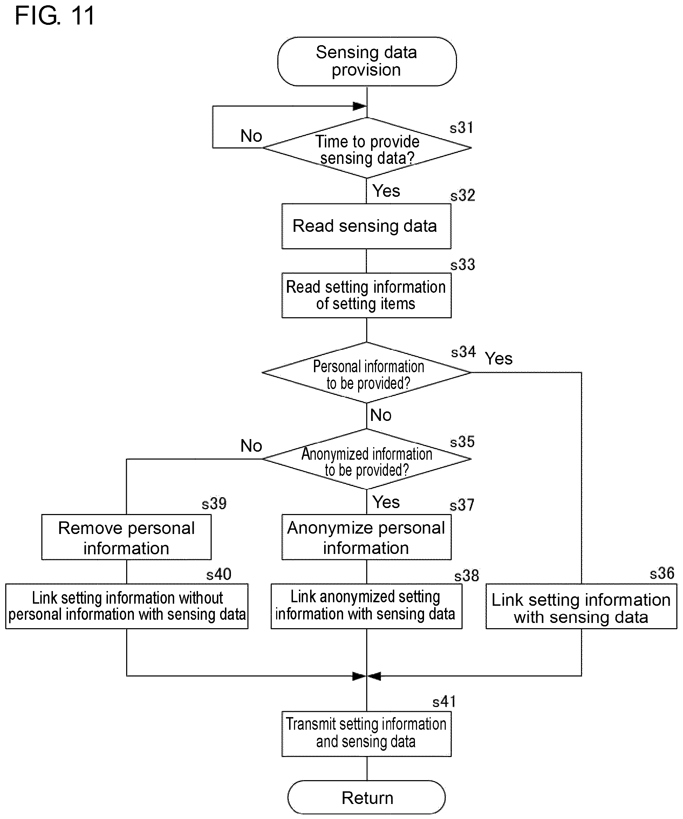

A process for providing sensing data performed by the GW terminal 1 will now be described. Fig. 11 is a flowchart showing the sensing data provision process. When detecting the time at which sensing data is to be provided to the application system 5, the sensing data output limit unit 25 in the GW terminal 1 reads sensing data to be provided from the sensing data storage DB 18 based on the first data catalog 100a in the provider data catalog 100 associated with the sensing data to be provided (s31 and s32). The time to provide the sensing data includes when the status of the sensing device 2 is changed, when any item in the provider data catalog 100 is updated, predetermined times (at regular or irregular intervals), and when the request for sensing data is provided from the user. In s32, sensing data with monitoring characteristics selected for provision to the first data catalog 100a in the provider data catalog 100 is read, whereas sensing data with monitoring characteristics unselected for provision is not read. More specifically, in s32, the sensing data output limit unit 25 performs filtering to read no sensing data with monitoring characteristics unselected for provision to the first data catalog 100a in the provider data catalog 100 from the sensing data storage DB 18.

The setting information link unit 26 reads, from the in-terminal personal information storage DB 19 and the external website information storage DB 20, the setting information of the setting items to be linked with the sensing data to be provided this time (s33). In s33, the setting information link unit 26 reads the setting information of the setting items selected for provision in the second data catalog 100b included in the provider data catalog 100, and does not read the setting information of the unselected setting items. More specifically, in s33, the setting information link unit 26 filters out the setting information representing the setting items unselected for provision in the second data catalog 100b in the provider data catalog 100.

The setting information link unit 26 determines whether the personal information is set for provision to the user (s34). When the personal information is not set for provision to the user, the setting information link unit 26 determines whether the anonymized information is set for provision to the user (s35). The setting information link unit 26 performs the determination in s34 and s35 by referring to the provider data catalog 100. As described above, the operator sets whether to provide the personal information to the user and whether to provide the anonymized information to the user when generating the provider data catalog 100.

When determining that the personal information is set for provision to the user, the setting information link unit 26 links the setting information of the setting items read in s33 with the sensing data read in s32 (s36). When determining that the anonymized information, instead of the personal information, is set for provision to the user, the setting information link unit 26 anonymizes the personal information included in the sensing data and the setting information of the setting items read in s32 and s33 (s37). The setting information link unit 26 then links the setting information including the anonymized personal information generated in s37 with the sensing data including the anonymized personal information generated in s37 (s38). When determining that neither the personal information nor the anonymized information is set for provision to the user, the setting information link unit 26 removes (deletes) the personal information from the sensing data and the setting information of the setting items read in s32 and s33 (s39). The setting information link unit 26 then links the setting information from which the personal information is removed in s39 with the sensing data from which the personal information is removed in s39 (s40).

The setting information link unit 26 transmits, to the application system 5, the setting information of the setting items and the sensing data linked with the setting information in any of steps s36, s38, and s40 (s41). The GW terminal 1 repeats the processing in s31 to s41. In s41, the setting information of the setting items and the sensing data linked with the setting information may be separately transmitted to the application system 5, or the setting information of the setting items may be incorporated into the sensing data linked with the setting information and then transmitted to the application system 5.

As described above, the GW terminal 1 according to the embodiment allows, through edits to the provider data catalog 100, easy setting of sensing data and personal information or other information unintended by the provider not to be distributed to the user. This structure reduces sensing data and personal information or other information unintended by the provider being distributed to the user.

The GW terminal 1 provides, to the application system 5, the setting information of the setting items together with the sensing data obtained by the sensing devices 2 or the internal sensors incorporated in the sensor unit 13. The application system 5 as the user can thus classify the monitoring target for the sensing data using the setting information of the setting items provided together with the sensing data. For example, when the monitoring target is a person, the monitoring target for the sensing data can be classified based on, for example, the height, age, sex, lifestyle pattern, and history of taking medicines (medication history). The user can thus obtain analysis results of the sensing data for each class of monitoring targets. More specifically, the user can obtain analysis results that are highly useful and usable for a wide range of services. In other words, the GW terminal 1 increases the usefulness of the sensing data to be provided to the application system 5 as the user.

The provider can set whether to provide, to the user, the personal information, the anonymized information generated by anonymizing the personal information, or neither the personal information nor the anonymized information as intended. The provider can thus manage the personal information appropriately.

In the above example, the GW terminal 1 transmits the setting information of the setting items to the application system 5 as the user in addition to the sensing data. In another example, the GW terminal 1 does not transmit the setting information of the setting items to the application system 5 as the user (or more specifically, transmits sensing data to the application system 5 without transmitting the setting information of the setting items). In this case, the GW terminal 1 may include no component for processing the setting information of the setting items.

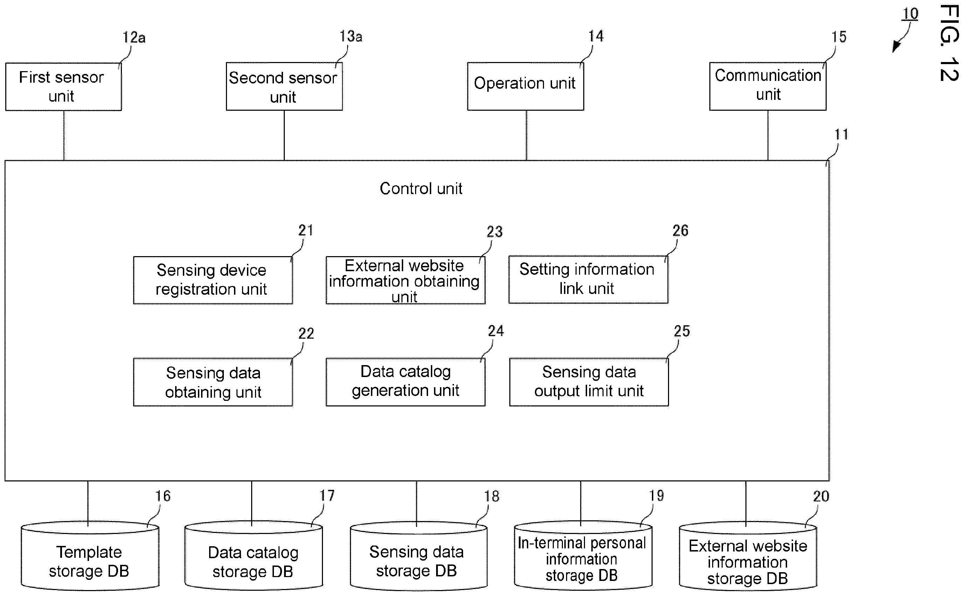

Fig. 12 is a block diagram of a sensor device according to another embodiment of the present invention showing its main components. A sensor device 10 according to the embodiment includes the structure equivalent to the GW terminal 1 described above and the structure equivalent to a sensing device 2 in an integrated manner (the sensor device 10 includes a sensing device 2 incorporating the structure equivalent to the GW terminal 1 described above).

In Fig. 12, the same components as in Fig. 2 are given the same reference numerals.

The sensor device 10 according to the embodiment includes the control unit 11, a first sensor unit 12a, a second sensor unit 13a, the operation unit 14, the communication unit 15, the template storage DB 16, the data catalog storage DB 17, the sensing data storage DB 18, the in-terminal personal information storage DB 19, and the external website information storage DB 20. Similarly to the GW terminal 1 described above, the control unit 11 includes the sensing device registration unit 21, the sensing data obtaining unit 22, the external website information obtaining unit 23, the data catalog generation unit 24, the sensing data output limit unit 25, and the setting information link unit 26. The sensor device 10 may communicate with external devices, such as the sensor network server 3, the external website 4, and the application system 5 described above, through a communication device not shown. In this case, the communication unit 15 in the sensor device 10 may perform near-field communication with the communication device, or include an interface connected to the communication device with wires for input and output operations.

The sensor device 10 in this embodiment includes the first sensor unit 12a in place of the sensing device connection unit 12 described above. The first sensor unit 12a includes one or more sensors. The sensing data obtaining unit 22 obtains the sensing data obtained by the sensors included in the first sensor unit 12a. The sensing data obtained by the sensors in the first sensor unit 12a is the sensing data to be provided to the application system 5 as the user. The first sensor unit 12a corresponds to a sensor in an aspect of the present invention.

The second sensor unit 13a shown in Fig. 12 has the same structure as the sensor unit 13 in the GW terminal 1 described above (the second sensor unit 13a is labeled as such to be distinguishable from the first sensor unit 12a in this example).

The sensor device 10 according to the embodiment may have the sensors in the first sensor unit 12a registered in default. In this case, the sensor device 10 may eliminate the registration shown in Fig. 5.

In the same manner as the GW terminal 1 described above, the sensor device 10 performs the data collection shown in Fig. 10 and the sensing data provision shown in Fig. 11. The sensor device 10 differs from the GW terminal 1 described above in sensing the characteristics of a monitoring target with the sensors included in the first sensor unit 12a, rather than receiving the sensing data from the sensing devices 2 connected to the sensing device connection unit 12. The sensor device 10 produces the same advantageous effects as the GW terminal 1 described above.

The present invention is not limited to the above embodiments, but the components may be modified without departing from the spirit and scope of the invention in its implementation. The components described in the above embodiments may be combined as appropriate to provide various aspects of the invention. For example, some of the components described in the above embodiments may be eliminated. Further, components in different embodiments may be combined as appropriate.

The above embodiments may be partially or entirely expressed in, but not limited to, the following forms shown in the appendixes below.

### Appendix 1

A sensor management unit, comprising:
at least one hardware processor configured to
obtain sensing data sensed by a sensor;
output, from an output unit, sensing data to be provided to a user based on a data catalog storing an attribute of the sensing data; and
determine data to be output from the output unit based on an item to be output to the user stored in the data catalog, and output the determined data.

### Appendix 2

A sensing data providing method implementable by at least one hardware processor, the method comprising:
obtaining sensing data sensed by a sensor;
outputting, from an output unit, sensing data to be provided to a user based on a data catalog storing an attribute of the sensing data; and
determining data to be output from the output unit based on an item to be output to the user stored in the data catalog, and outputting the determined data.

### REFERENCE SIGNS LIST

1 gateway terminal (GW terminal)
2 sensing device
3 sensor network server
4 external website
5 application system
6 network
10 sensor device
11 control unit 12
sensing device connection unit
12a first sensor unit
13 sensor unit
13a second sensor unit
14 operation unit
15 communication unit
16 template storage database (template storage DB)
17 data catalog storage database (data catalog storage DB)
18 sensing data storage database (sensing data storage DB)
19 in-terminal personal information storage database (in-terminal personal information storage DB)
20 external website information storage database (external website information storage DB)
21 sensing device registration unit
22 sensing data obtaining unit
23 external website information obtaining unit
24 data catalog generation unit
25 sensing data output limit unit
26 setting information link unit
50 information collection table
100 provider data catalog
100a first data catalog
100b second data catalog
101 user data catalog

## Claims

1. A sensor management unit, comprising:
a sensing data obtaining unit configured to obtain sensing data generated by a sensor;
an output determination unit configured to determine whether the sensing data is allowed to be output to a user based on a data catalog (100) storing an attribute of the sensing data and storing a first item allowed to be output to the user; and
an output unit configured to output the sensing data to the user in accordance with a result of the determination by the output determination unit.

2. The sensor management unit according to claim 1, wherein
the output determination unit further determines whether metadata about the sensing data is allowed to be output to the user based on the data catalog (100), and
the output unit further outputs the metadata to the user in accordance with a result of the determination.

3. The sensor management unit according to claim 1 or claim 2, further comprising:
an edit unit configured to receive an edit to the first item.

4. The sensor management unit according to claim 3, wherein
the edit unit receives an edit indicating whether to set an item of the sensing data as the first item.

5. The sensor management unit according to claim 2, further comprising:
an edit unit configured to receive an edit to the first item,
wherein the metadata includes personal information about a provider, and
the edit unit receives an edit indicating whether to set the personal information as the first item.

6. The sensor management unit according to claim 2, wherein
the data catalog (100) is configured to store a second item allowed to be output to the user after conversion into anonymized information,
the sensor management unit further comprises an edit unit configured to receive an edit to the second item,
the metadata includes personal information about a provider, and
the edit unit receives an edit indicating whether to set the personal information as the second item.

7. A sensor device (10), comprising:
the sensor management unit according to any one of claims 1 to 6; and
the sensor.

8. A sensing data providing method implementable by a computer, the method comprising:
obtaining sensing data generated by a sensor;
determining whether the sensing data is allowed to be output to a user based on a data catalog (100) storing an attribute of the sensing data and storing an item allowed to be output to the user; and
outputting the sensing data to the user in accordance with a result of the determination in the determining whether the sensing data is allowed to be output to a user.

9. A sensing data providing program, causing a computer to implement:
obtaining sensing data generated by a sensor;
determining whether the sensing data is allowed to be output to a user based on a data catalog (100) storing an attribute of the sensing data and storing an item allowed to be output to the user; and
outputting the sensing data to the user in accordance with a result of the determination in the determining whether the sensing data is allowed to be output to a user.
